# FASCICULE DE BREVET EUROPEEN

(11) **EP 1 696 935 B1**
(45) Date de publication et mention de la délivrance du brevet: **04.04.2007**
(21) Numéro de dépôt: 04816470.1
(22) Date de dépôt: 22.12.2004
(51) Int. Cl.: A61K 31/706, C07H 17/00

(54) **NOUVEAUX DERIVES DE LA MORPHINE-6-GLUCURONIDE, COMPOSITIONS PHARMACEUTIQUES LES CONTENANT, LEUR PROCEDE DE PREPARATION ET LEURS UTILISATIONS**
NEUE DERIVATE VON MORPHIN-6-GLUCURONID, DIESE DERIVATE ENTHALTENDE PHARMAZEUTISCHE ZUSAMMENSETZUNGEN, HERSTELLUNGS- UND VERWENDUNGSVERFAHREN DAFÜR
NOVEL DERIVATIVES OF MORPHINE-6-GLUCURONIDE, PHARMACEUTICAL COMPOSITIONS CONTAINING SAID DERIVATIVES, PREPARATION METHOD THEREOF AND USES OF SAME

(30) Priorité: 22.12.2003 FR 0315160
(43) Date de publication de la demande: 06.09.2006
(73) Titulaire: CLL PHARMA, 06200 Nice (FR)
(72) Inventeur: TEMSAMANI, Jamal, F-30900 Nîmes (FR); LAHANA, Roger, F-30900 Nîmes (FR); MOUCHET, Patrick, F-30300 Fourques (FR)
(74) Mandataire: Ulmann, Catherine Claire
(86) Numéro de dépôt international: PCT/FR2004/003342
(87) Numéro de publication internationale: WO 2005/063263

(56) Documents cités:
- EP-A- 0 816 375
- FR-A- 2 821 272

## Description

La présente invention a pour objet de nouveaux dérivés de la morphine 6-glucuronide, leur procédé de préparation ainsi que leurs utilisations en thérapie, notamment en tant qu'analgésiques.

La morphine est actuellement l'analgésique le plus utilisé dans le traitement des douleurs de moyenne et de grande intensité. On distingue au niveau du système nerveux central trois classes principales de récepteurs opioïdes : µ (mu), κ (kappa) et δ (delta). La morphine, de même que d'autres opioïdes, produisent leurs principaux effets sur le système nerveux central et le système digestif par l'intermédiaire des récepteurs µ-opioïdes. Il existe deux sous-types de récepteurs µ: le type µ1 de très haute affinité et faible capacité, et le type µ2 de basse affinité et forte capacité (Pasternak & Wood, 1986. *Life Sci* 38 :1889-1898). La liaison aux récepteurs µ1 entraîne une réaction analgésique de type supraspinal et la diminution du *turnover* de l'acétylcholine, tandis que la liaison aux récepteurs µ2 entraîne une réaction analgésique de type spinal et est responsable de la dépression respiratoire et de l'inhibition du transit intestinal.

Les mécanismes par lesquels la morphine exerce son action analgésique ne sont pas encore complètement élucidés. On sait qu'elle subit un important métabolisme, qui conduit à des métabolites dont certains contribuent à son action analgésique. Le foie apparaît comme le site principal de sa biotransformation. La morphine subit principalement une glucuronidation énantio-sélective catalysée par l' UDP-glucuronyltransférase (UGT) qui conduit à la formation de deux métabolites : la morphine-3-glucuronide (ci-après également désignée « M3G ») et la morphine-6-glucuronide (ci-après également désignée « M6G »).

Il a été démontré que la modification de la position 3-hydroxy de la morphine diminuait l'activité analgésique, alors que des modifications de la position 6-hydroxy peuvent, au contraire, augmenter l'activité analgésique (Aderjan & Skopp, 1998, Therapeutic Drug Monitoring 20 :561-569).

Ainsi, la M3G n'a pas d'affinité pour les récepteurs opioïdes et ne participe pas à l'activité analgésique de la morphine.

En revanche, la morphine-6-glucuronide a une forte affinité pour les récepteurs opioïdes et il a été démontré qu'elle a un effet analgésique aussi bien chez les rongeurs que chez l'homme.

La M6G a été décrite comme étant un analgésique plus puissant que la morphine elle-même après une administration centrale (Paul et al, 1989. J Pharmacol. Exp. Ther. 49 ; 6280-6284 ; Frances et al, 1992. J Pharmacol. Exp. Ther. 262 ; 25-31) et ayant la même activité par voie systémique. Des études de liaison ligand-récepteur aux opiacés réalisées in vitro ont montré que la M6G se liait aux récepteurs opioïdes et qu'elle était de 1 à 5 fois moins affine pour les récepteurs µ que la morphine (Christensen & Jorgensen 1987. Pharmacol Toxicol.60 :75-76; Frances et al, 1992 J Pharmacol. Exp. Ther. 262 ; 25-31).

D'autres métabolites de la morphine, en particulier la normorphine, ont montré une certaine activité analgésique. Cependant, ces autres métabolites sont présents en de faibles concentrations et ne sont pas susceptibles de contribuer de manière significative à l'effet global de la morphine.

Toutefois, malgré sa grande efficacité, le traitement de la douleur par la morphine s'accompagne d'effets secondaires indésirables tels que : dépression respiratoire, inhibition du transit intestinal, nausées, vomissements, et surtout syndrome de dépendance et induction de tolérance.

On a donc cherché à mettre au point d'autres substances actives, présentant une efficacité analgésique comparable à la morphine, mais n'ayant pas tout ou partie de ses effets secondaires indésirables.

Bien entendu, en raison de son activité analgésique exposée plus haut, on a proposé d'utiliser la M6G en tant que substitut de la morphine.

On peut, à cet égard, faire référence à la demande internationale WO 95/05831 visant l'utilisation d'une composition pharmaceutique pour administration orale, contenant de la M6G, pour le traitement de la douleur.

La demande internationale WO 99/64430 décrit une méthode pour la synthèse de la M6G et de ses intermédiaires. Le brevet US 5,621,087 décrit un nouveau procédé pour la préparation de la M6G ou de certains de ses dérivés.

La M6G, qui, on l'a vu, présente des propriétés analgésiques comparables à la morphine, a pour avantage de diminuer nausées et vomissements. Toutefois, la M6G ne contribue pas à la suppression d'autres effets indésirables de la morphine, à savoir la dépression respiratoire et le syndrome de dépendance (Osborne et al, 1992. *Br. J*. *Clin. Pharmac* 34 :130-138). Les brevets FR 2821272, et EP 0816375 décrivent egalement des derivés de la M6G comme analgésiques.

Le brevet US 6,150,524 décrit des procédés pour la synthèse d'autres dérivés de la morphine, qui sont dits présentés des propriétés analgésiques fortes et qui peuvent être administrés par voie orale.

Il est également connu que l'association d'un composé se liant aux récepteurs µ et d'un composé se liant aux récepteurs κ, présente un effet analgésique puissant sans les effets secondaires de dépendance physique et de dépression respiratoire (Rothman et al 2000 ; J Subst Abuse Treat 19 :277-281 ; Shook et al, 1990 Am Rev Respir Dis 142 :895-909).

Cependant, à la connaissance des inventeurs, il n'existe pas d'analgésique d'une efficacité comparable à celle de la morphine ou de la M6G, mais qui ne présente pas, ou moins, d'effets secondaires, notamment en ce qui concerne la dépendance physique et la dépression respiratoire.

La présente invention a alors pour principal objet de nouveaux composés, dérivés de la M6G, qui permettent de résoudre ce problème. Plus particulièrement, les composés de l'invention présentent l'avantage de posséder une affinité pour les récepteurs κ plus grande que la M6G sans pour autant présenter une affinité réduite pour les récepteurs µ afin d'obtenir un composé ayant une activité analgésique puissante mais moins d'effets secondaires,

Dans le cadre de leurs travaux de recherche, les inventeurs ont ainsi pu déterminer que la modification de la M6G par substitution à l'aide d'un groupement porteur d'une fonction thiol ou d'un atome de soufre permet d'augmenter de façon significative l'affinité pour les récepteurs κ sans pour autant diminuer celle pour les récepteurs µ.

L'invention concerne ainsi un composé de formule (A) : 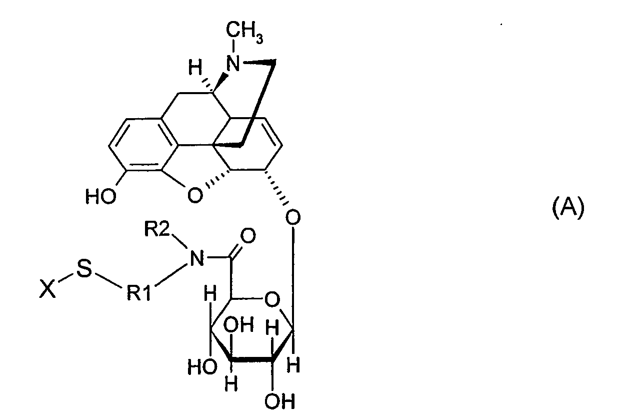 dans laquelle :
- l'ensemble de l'entité ci-dessus, à l'exception du substituant X, est dénommé M6G-N(R₂)R₁-S-
- R₁ représente un groupe alkyle linéaire ou ramifié en C₁-C₁₀, non substitué ou substitué par au moins un substituant, la chaîne alkyle étant éventuellement interrompue par un ou plusieurs hétéroatomes choisis parmi O, S et N ;
- R₂ représente l'hydrogène, un groupe alkyle linéaire ou ramifié en C₁-C₅ ou un groupe aryle, hétéroaryle ou (C₁-C₅ )alkylaryle, non substitué ou substitué par un alkyle en C₁-C₄ ;
- X représente l'hydrogène, un résidu M6G-N(R₂)R₁-S- ou un polymère lié au reste de l'entité par un bras espaceur ;
- les carbones asymétriques présents dans la formule (A) peuvent être de configuration R ou S.

Lorsque R₁ représente un groupe alkyle substitué par un ou plusieurs substituants, le (ou les) substituant(s) est (sont) par exemple choisi(s) parmi : un groupe alkyle en C₁-C₅; un groupe amino ; un groupe COOR₃; un groupe CONR₃R₄, R₃ et R₄ dans les groupes COOR₃ ou CONR₃R₄ représentant indépendamment l'hydrogène, un groupe alkyle en C₁-C₂₀ éventuellement substitué, aryle, hétéroaryle ou alkylaryle; une cétone en C₁-C₂₀, de préférence en C₁-C₁₀ ; un aldéhyde en C₁-C₂₀, de préférence en C₁-C₁₀.

Lorsque R₂ représente un groupe aryle ou hétéroaryle monocyclique, celui-ci peut par exemple être choisi parmi les groupes phényle, thiophényle, pyridyle, pyrrolyle, pyrazolyle, furanyle, ou indolyle . Lorsque R₂ représente un groupe alkylaryle, celui-ci peut par exemple être le benzyle.

Des composés préférés aux fins de l'invention sont les composés de formule (A) dans laquelle R₁ représente un groupe alkyle linéaire ou ramifié en C₁-C₁₀, en particulier méthyle, éthyle, propyle ou butyle, non substitué ou substitué par au moins un substituant, la chaîne alkyle étant éventuellement interrompue par un ou plusieurs hétéroatomes choisis parmi O, S et N, R₂ représente l'hydrogène et X représente l'hydrogène .

Parmi ceux-ci, le composé de formule (A) dans laquelle R₁ représente -(CH₂)₂- , R₂ est l'hydrogène et X est l'hydrogène est préféré. Un tel composé représenté dans la structure (I) ci-dessous est appelé M6G-Cystéamide.

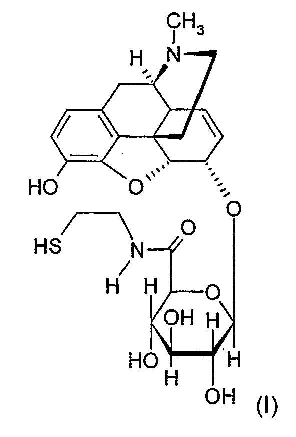

Des composés préférés sont ceux dans lesquels X représente un résidu M6G-N(R₂)R₁-S-. Dans ce cas, la structure (A) correspond à la forme oxydée de la structure (A) initiale, et se trouve dans ce cas sous forme de dimère.

Les deux résidus M6G-N(R₂)R₁-S- constituant les composés de formule (A) sous forme de dimère peuvent être identiques ou différents.

Des composés de ce type particulièrement avantageux sont ceux dans lesquels les deux résidus M6G-N(R₂)R₁-S- sont identiques, et les composés dimères ont une structure symétrique.

Il a été montré dans la littérature que les liaisons disulfure, relativement stables dans le plasma, peuvent être clivées à l'intérieur des cellules pour redonner une fonction thiol, et de ce fait pourrait permettre d'améliorer les propriétés des molécules actives in vivo (G. Saito et al., Advanced Drug Delivery Reviews, 2003, 55, 199-215).

Des composés préférés selon l'invention sont des composés de formule (A) dans laquelle R₁ est tel que défini plus haut, R₂ est l'hydrogène et X est un résidu M6G-N(R₂)R₁-S- tel que défini plus haut.

Un composé préféré de formule (A) dans laquelle R₁ représente -(CH₂)₂- , R₂ est l'hydrogène et X est un résidu M6G-N(R₂)R₁-S- dans lequel R₁ = (CH₂)₂ et R₂ est l'hydrogène, représenté dans la structure (II) ci-dessous est appelé M6G-Cya-Cya-M6G. Le composé (II) est la forme oxydée, donc dimérisée, du composé (I).

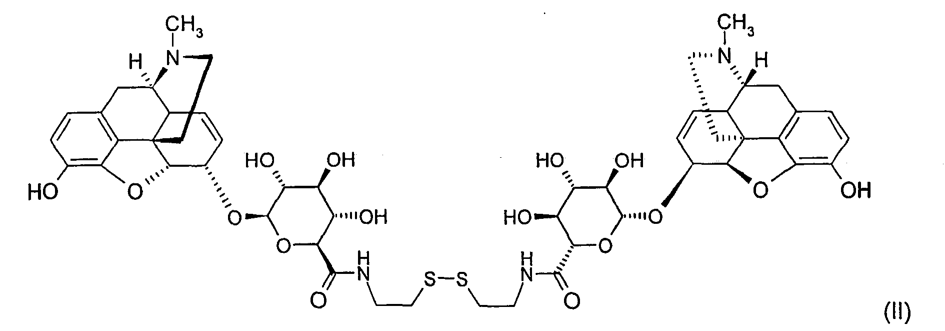

D'autres composés avantageux de formule (A) sont, par exemple, ceux dans lesquels :
- R₁ représente un groupe -CH(COOR₃)-CH₂- dans lequel R₃ représente l'hydrogène ou un alkyle, en particulier méthyle, éthyle, propyle ou butyle, R₂ représente l'hydrogène et X représente l'hydrogène ou un résidu M6G-N(R₂)R₁-S- dans lequel R₁ = -CH(COOR₃)-CH₂- dans lequel R₃ est tel que défini ci-dessus et R₂ est l'hydrogène;
- R₁ représente un groupe -CH(CONR₃R₄)-CH₂- dans lequel R₃ et R₄ représente l'hydrogène ou un alkyle, en particulier méthyle, éthyle, propyle
ou butyle, R₂ représente l'hydrogène et X représente l'hydrogène ou un résidu M6G-N(R₂)R₁-S- dans lequel R₁ = -CH(CONR₃R₄)-CH₂- dans lequel R₃ et R₄ sont tels que défini ci-dessus et R₂ est l'hydrogène;
- R₁ représente un groupe -CH(COOR₃)-C(CH₃)₂- dans lequel R₃ représente l'hydrogène ou un alkyle, en particulier méthyle, éthyle, propyle ou butyle, R₂ représente l'hydrogène et X représente l'hydrogène ou un résidu M6G-N(R₂)R₁-S- dans lequel R₁ = -CH(COOR₃)-C(CH₃)₂- dans lequel R₃ est tel que défini ci-dessus et R₂ est l'hydrogène;
- R₁ représente un groupe -CH(COOR₃)-(CH₂)₂-C(O)NHCH(R₅)-CH₂-, dans lequel R₃ représente hydrogène ou un alkyle, en particulier méthyle, éthyle, propyle ou butyle, R₅ représente -C(O)-NH-CH₂-COOR₃ dans lequel R₃ est tel que défini ci-dessus, R₂ représente l'hydrogène et X représente l'hydrogène ou un résidu M6G-N(R₂)R₁-S- dans lequel R₁ = -CH(COOR₃)-(CH₂)₂-C(O)NHCH(R₅)-CH₂- dans lequel R₃ et R₅ sont tels que définis ci-dessus et R₂ représente l'hydrogène.

D'autres composés intéressants sont ceux dans lesquels X représente un polymère lié au reste de l'entité par un bras espaceur.

En effet, il a été montré dans la littérature que la conjugaison d'une molécule organique d'intérêt biologique avec un poly(éthylène glycol) permettait d'augmenter la demi-vie plasmatique de cette molécule (R.B. Greenwald et al., Advanced Drug Delivery Reviews, 2003, 55, 217-250).

De préférence, on utilisera un bras espaceur ayant la formule S-(CH₂)ₙ-NH-C(O)- dans laquelle n=0 à 4, de préférence 2.

On peut également utiliser d'autres types de bras espaceurs, tels que par exemple un radical organique bivalent, choisi parmi les groupes alkylène linéaires ou ramifiés en C₁-C₂₀ contenant éventuellement une ou plusieurs doubles liaisons ou triples liaisons et :ou contenant éventuellement un ou plusieurs hétéroatomes tels que O, N, S, P, ou un ou plusieurs groupe(s) carbamoyle ou carboxamido ; les groupes cycloalkylène en C₅-C₈ et les groupes arylène en C₆-C₁₄, lesdits groupes alkylène, cycloalkylène ou arylène étant éventuellemnt substitués par des groupes alkyle, aryle ou sulfonate.

Parmi les composés dans lesquels X représente un polymère lié au reste de l'entité par un bras espaceur, les composés de formule (A) dans lesquels R₁ représente un groupe -(CH₂)₂- , R₂ représente l'hydrogène et X représente un polymère lié au reste de l'entité par un bras espaceur de formule -S-(CH₂)ₙ-NH-C(O)- dans laquelle n= 0 à 4, de préférence 2, et ledit polymère est un poly(éthylène glycol) (également dénommé PEG) de poids moléculaire (Mw) supérieur ou égal à 10000, sont des composés préférés selon l'invention .

L'invention concerne également, selon un aspect ultérieur, un procédé pour la préparation des composés de formule (A).

Ledit procédé comprend les étapes consistant à faire réagir la morphine-6-glucuronide avec un composé de formule (III) NHR₂-R₁-S-S-R₁-NHR₂, dans laquelle R₁ et R₂ sont tels que définis ci-dessus, en présence d'un agent de couplage et à réduire *in situ* le pont disulfure à l'aide d'un agent réducteur si nécessaire (c'est-à-dire lorsque X= H dans la formule (A)).

De préférence, la réaction de la morphine-6-glucuronide avec le composé de formule (III) a lieu en milieu basique.

Pour la préparation des composés de formules (I) ou (II), on utilisera par exemple un composé de formule (III) dans laquelle R₂ est l'hydrogène et R₁ représente un groupe -(CH₂)₂-, dénommé cystamine .

A titre d'agent de couplage, on peut citer les agents de couplages habituellement utilisés en synthèse peptidique, tels que le benzotriazol-1-yl-oxy-tris-pyrrolidino-phosphonium hexafluorophosphate (PyBOP), la dicyclohexylcarbodiimide (DCC), la DCC associée à l'hydroxybenzotriazole (DCC/HOBT) ou la diisopropylcarbodiimide associée à l'HOBT (DIPCDI/ HOBT).

On utilisera de préférence l'agent de couplage en excès molaire d'environ 1,1 à 4 équivalents molaires pour 1 équivalent molaire de composé de formule (III).

Le couplage est de préférence réalisé à température ambiante, dans un solvant polaire tel que par exemple le diméthylformamide (DMF), la N-méthylpyrrolidone (NMP), le dichlorométhane ou l'acétonitrile.

A titre d'agent réducteur, on peut citer par exemple la tris(2-carboxyéthyl)phosphine, la triphénylphosphine, la tris(hydroxyméthyl)-phosphine ou le dithiothréitol.

On utilisera de préférence l'agent réducteur en excès molaire d'environ 1,1 à 5 équivalents.

La réduction a lieu de préférence à température ambiante et à un pH inférieur à 7.

Selon un autre aspect de l'invention, le composé de formule (A) dans laquelle X = H peut être obtenu par un procédé comprenant les étapes consistant à faire réagir la morphine-6-glucuronide avec un composé de formule (IV) NHR₂-R₁-SH, dans laquelle R₁ et R₂ sont tels que définis ci-dessus, en présence d'un agent de couplage et à réduire *in situ* les sous-produits d'oxydation à l'aide d'un agent réducteur.

De préférence, la réaction de la morphine-6-glucuronide avec le composé de formule (IV) a lieu en milieu basique.

On peut notamment utiliser un composé de formule (IV) dans laquelle R₂ est l'hydrogène et R₁ représente un groupe -(CH₂)₂-, dénommé cystéamine. A titre d'exemples de composés de formule (IV), on peut également citer la cystéine méthyl ester, pénicillamine ou la glutathione.

A titre d'agent de couplage, on peut utiliser les agents de couplages habituellement utilisés en synthèse peptidique, tels que ceux cités ci-dessus.

On utilisera de préférence l'agent de couplage en excès molaire d'environ 1,1 à 2 équivalents molaires pour 1 équivalent molaire de morphine-6-glucuronide.

Le couplage est de préférence réalisé à température ambiante, dans un solvant polaire tel que par exemple le diméthylformamide (DMF), la N-méthylpyrrolidone (NMP), le dichlorométhane ou l'acétonitrile.

L'agent réducteur peut être choisi parmi les agents réducteurs habituellement utilisés en chimie peptidique tels que ceux cités ci-dessus.

On utilisera de préférence l'agent réducteur en quantité d'environ 0,5 à 5 équivalents molaires.

La réduction a lieu de préférence à température ambiante et à un pH inférieur à 7.

L'invention concerne également une composition pharmaceutique contenant à titre de principe actif un composé de formule (A) tel que décrit ci-dessus ou un de ses sels pharmaceutiquement acceptables, et au moins un véhicule pharmaceutiquement acceptable.

Par " sel pharmaceutiquement acceptable", on entend par exemple et de manière non limitative un acétate, un sulfate ou un chlorhydrate.

Avantageusement, la composition pharmaceutique selon l'invention se présentera sous une forme appropriée pour une administration :
- par voie parentérale, comme par exemple, sous forme de préparations injectables par voie sous-cutanée, intraveineuse ou intramusculaire;
- par voie orale, comme par exemple, sous forme de comprimés enrobés ou non, de gélules, de poudres, de granulés, de suspensions ou de solutions orales. Une telle forme pour l'administration par voie orale peut être soit à libération immédiate, soit à libération prolongée ou retardée. De telles formes à libération prolongée ou retardée sont décrites, par exemple, dans les demandes EP 253 104 ou EP 576 643 ;
- par voie rectale, comme par exemple, sous forme de suppositoires ;
- par voie topique, notamment transdermique, comme par exemple, sous la forme de " patch ", de pommade ou de gel.
- par voie intranasale, comme par exemple sous forme d'aérosols et "sprays ",
- par voie perlinguale,
- par voie intraoculaire.

Le véhicule pharmaceutiquement acceptable peut être choisi parmi les véhicules utilisés de manière classique selon chacun des modes d'administration.

L'invention concerne également l'utilisation d'un composé de formule (A) ou d'un de ses sels pharmaceutiquement acceptables pour la fabrication d'un médicament utile pour le traitement de la douleur, en particulier pour le traitement de douleurs aiguës ou des douleurs chroniques, de douleurs neuropathiques, musculaires, osseuses, post-opératoires, de la migraine, les douleurs du cancer, les lombalgies, les douleurs arthrosiques, les douleurs associées au diabète ou les douleurs associées au SIDA.

L'invention est illustrée de manière non limitative par les exemples ci-dessous.

### Exemples :

### A. synthèses

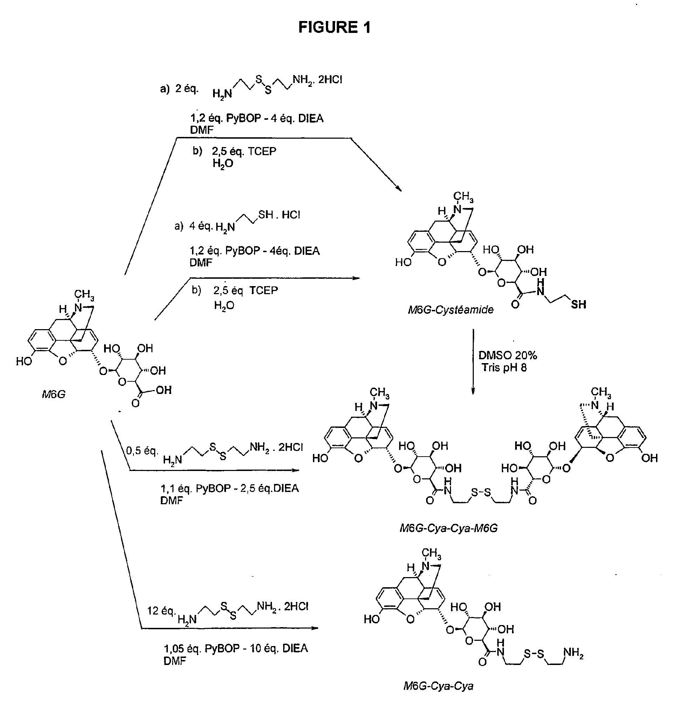

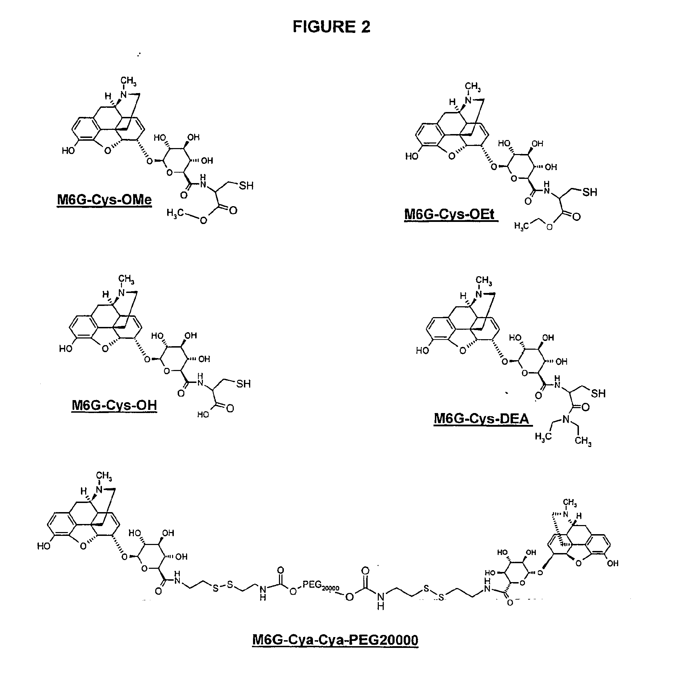

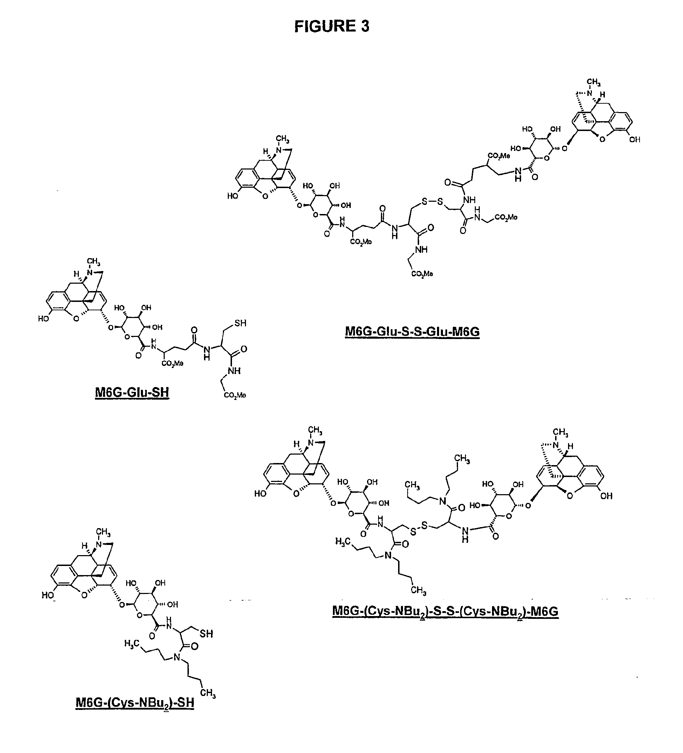

Les réactions ont été suivies par chromatographie liquide haute pression (HPLC) analytique en phase inverse et Spectrométrie de Masse (MS). Les puretés et l'identité des composés obtenus sont confirmées par HPLC analytique en phase inverse et par spectrométrie de masse. Les différentes voies de synthèse sont réalisées selon le schéma représenté sur la figure 1. Les structures des composés synthétisés sont représentées sur les figures 1 à 3.

### Exemple 1 : synthèse de la M6G-Cystéamide

Les différentes voies de synthèse sont réalisées selon le schéma représenté sur la figure 1.

### Synthèse par couplage avec la cystéamine

Dans un réacteur (tube falcon) on a introduit 4 équivalents molaires de cystéamine sous sa forme chlorhydrate dans du diméthylformamide (DMF) à 200 g/l et 4 équivalents molaires de diisopropyléthylamine (DIEA). On a additionné 1 équivalent molaire de M6G en poudre, 1,2 équivalents molaires de benzotriazole-1-yl-oxy-tris-pyrrolidino-phosphonium hexafluorophosphate (PyBOP) préalablement solubilisé dans du DMF à 680 g/l, puis on a vérifié que le pH était toujours basique. On a laissé réagir pendant 3 h sous agitation à température ambiante.

On a alors réduit le pont disulfure des sous-produits d'oxydation générés lors du couplage en milieu basique, par la Tris(2-carboxyéthyl)phosphine sous sa forme chlorhydrate (TCEP) (0,72 équivalent molaire) à 150 g/l dans un mélange (acétonitrile/H₂O (50/50), acide trifluoroacétique 0,1%). Après 12 h de réaction sous agitation, le produit brut est alors purifié par HPLC préparative .
On obtient après lyophilisation le produit M6G-Cystéamide : [M+H]⁺ = 521,4 - M_{sel TFA =} 634 - Rdt = 66% - Pureté 95%.

### Synthèse par couplage avec la cystamine

Dans un réacteur (tube falcon) on a introduit 2 équivalents molaires de cystamine sous sa forme chlorhydrate dans du DMF à 30 g/l, puis on a ajouté 1 équivalent molaire de M6G dihydrate et 4 équivalents molaires de DIEA. On a vérifié que le pH était basique (≥ 9). On a additionné goutte à goutte en refroidissant par un bain de glace, 1,2 équivalents molaires de PyBOP préalablement solubilisé dans du DMF à 230 g/l. On a laissé réagir pendant 3 h sous agitation. On a alors réduit le pont disulfure par la Tris(2-carboxyéthyl)phosphine (2,5 équivalents molaires) à 215 g/l dans (H₂O acide trifluoroacétique 0,1%). Après 30 min de réaction sous agitation, le produit brut est alors purifié par HPLC préparative.
On obtient après lyophilisation le produit M6G-Cystéamide : [M+H]⁺ = 521,2 - M_{sel TFA} = 634 - Rdt = 93% - Pureté 98%.

### Exemple 2 : synthèse de la M6G-Cya-Cya-M6G

Les différentes voies de synthèse sont réalisées selon le schéma représenté sur la figure 1.

### Synthèse par couplage avec la cystamine

Dans un réacteur (tube falcon) on a solubilisé 1 équivalent molaire de cystamine sous sa forme chlorhydrate dans du DMF à 15 g/l, puis on a ajouté 5 équivalents molaires de DIEA et 2 équivalents molaires de M6G dihydrate. On a additionné goutte à goutte en refroidissant par un bain de glace, 2,4 équivalents molaires de PyBOP préalablement solubilisé dans du DMF à 230 g/1. On a laissé réagir pendant 12 h sous agitation. Le produit brut est purifié par HPLC .
On obtient après lyophilisation le produit M6G-Cya-Cya-M6G: [M+H]⁺ = 1035,7 - M_{sel TFA} = 1266 - Rdt = 80% - Pureté 98%.

### Synthèse par oxydation de la M6G-Cystéamide

Dans un réacteur (tube falcon) on a solubilisé la M6G-Cystéamide dans une solution de (diméthylsulfoxyde 20% - tampon aqueux Tris 200 mM pH=₈) à 90 g/l, puis on a laissé réagir pendant 48 h sous agitation. Le produit brut est purifié par HPLC .

On a obtenu, après lyophilisation, le produit M6G-Cya-Cya-M6G : [M+H]⁺ = 1040,1 - M_{sel TFA} = 1266 - Rdt = 95 % - Pureté 99%.

### Exemple 3 : synthèse de la M6G-Cya-Cya-PEG20000

### Couplage avec la cystamine

La synthèse de M6G-Cya-Cya est réalisée selon le schéma 1. Dans un réacteur (tube falcon) on a introduit 12 équivalents molaires de cystamine sous sa forme chlorhydrate dans du DMF à 65 g/l, puis on a ajouté 10 équivalents molaires de DIEA et 1 équivalent molaire de M6G dihydrate. On a ajouté 1,05 équivalents molaires de PyBOP et agité vigoureusement pendant 5 minutes. On a laissé réagir pendant 1 h sous agitation. Le produit brut est purifié par HPLC.
On a obtenu, après lyophilisation, le produit M6G-Cya-Cya: [M+H]⁺ = 596,3 - M_{sel TFA} = 823 - Rdt = 69% - Pureté 98%.

### Couplage au PEG20000

Dans un ballon, on a introduit 1 équivalent molaire de polyéthylène glycol 20000 dans du toluène à 20 g/l, puis on a ajouté 23 équivalents molaires de 4-nitrophényl chloroformate et de DIEA. On a chauffé pendant 12 h à 55°C. Le produit brut est purifié par cristallisation dans dichlorométhane/ éther diéthylique. On a obtenu le 4-nitrophényl-PEG-carbonate avec un rendement de l'ordre de 95%. Dans un ballon, on a introduit 1 équivalent molaire de 4-nitrophényl-PEG-carbonate dans du dichlorométhane à 250 g/l, puis on a ajouté 2 équivalents molaires de M6G-Cya-Cya dans du DMF à 40 g/l et 5 équivalents molaires de DIEA. On a agité pendant 12 h, précipité le produit brut à l'éther diéthylique puis purifié par cristallisation dans dichlorométhane/ éther diéthylique .

On a obtenu le M6G-Cya-Cya-PEG20000 avec un rendement de l'ordre de 85%.

### Exemple 4 : synthèse de la M6G-Cys-OEt

Dans un réacteur (tube falcon) on a introduit 4 équivalents molaires de cystéine éthyl ester sous sa forme chlorhydrate dans du DMF à 100 g/l et 4 équivalents molaires de DIEA. On a additionné 1 équivalent molaire de M6G en poudre, 1,05 équivalents molaires de PyBOP préalablement solubilisé dans du DMF à 109 g/l. On a laissé réagir pendant 2 h sous agitation.

On a alors réduit le pont disulfure des sous-produits d'oxydation, générés lors du couplage en milieu basique par 2 équivalents molaires de TCEP à 30 g/l dans un mélange (acétonitrile/H₂O-acide trifluoroacétique 0,1 % 1/1). Après 1 nuit de réaction sous agitation, le produit brut est alors purifié par HPLC préparative .
On obtient après lyophilisation le produit M6G-Cys-OEt: [M+H]⁺ = 593,5 - M_{sel TFA} = 706 - Rdt = 87% - Pureté 98%.

### Exemple 5 : synthèse de la M6G-Cys-DEA

Dans un réacteur (tube falcon) on a introduit 1 équivalent molaire de cystéine diéthyl amide sous sa forme trifluoroacétate dans du DMF à 77 g/l et 4 équivalents molaires de DIEA. On a additionné 1 équivalent molaire de M6G dihydrate en poudre, puis 1,05 équivalents molaires de PyBOP. On a laissé réagir pendant 1 h sous agitation.

On a alors réduit le pont disulfure des sous-produits d'oxydation par 2 équivalents molaires de TCEP à 30 g/l dans un mélange (H₂O-acide trifluoroacétique 0,1%). Après 1 h de réaction sous agitation, le produit brut est purifié par HPLC préparative . On obtient après lyophilisation le produit M6G-Cys-DEA: [M+H]⁺ = 620,2 - M_{sel TFA} = 733 - Rdt = 93% - Pureté 98%.

### Exemple 6: synthèse de M6G-Glu-S-S-Glu-M6G

### Estérification des fonctions acides de la glutathione:

Dans un réacteur (réacteur Wheaton) on introduit 1 équivalent molaire de glutathione oxydée dans du méthanol à 100 g/l et 0,1 équivalent molaire d'acide sulfurique. On chauffe le milieu réactionnel à 80 °C sous agitation pendant 4 h, puis l'agitation est maintenue 15 h à température ambiante et le milieu est de nouveau chauffé à 80°C pendant 6 h. Le produit brut est alors purifié par HPLC préparative.
On obtient après lyophilisation la glutathione oxydée estérifiée: [M+H]⁺ = 669 - M_{sel TFA} = 896 - Rdt = 17% - Pureté 76%.

### Couplage de la M6G :

Dans un réacteur (réacteur Wheaton) 2 équivalents molaires de M6G dihydrate en poudre sont mis en suspension dans du DMF à 93 g/l. On ajoute 1 équivalent molaire de glutathione oxydée estérifiée, 2 équivalents molaires de PyBOP et 4 équivalents molaires de DIEA. On laisse réagir 5 h sous agitation à température ambiante. Le milieu réactionnel est alors purifié par HPLC préparative.
Après lyophilisation on obtient le dimère M6G-Glu-S-S-Glu-M6G: [M+H]⁺= 1555 - M_{sel TFA} = 1782 - Pureté 91%.

### Exemple 7 : synthèse de la M6G-Glu-SH

### Réduction du dimère M6G-Glu-S-S-Glu-M6G:

Dans un réacteur (tube falcon) on introduit 1 équivalent molaire de dimère M6G-Glu-S-S-Glu-M6G et 3 équivalents molaires de TCEP à 88 g/l d'un mélange acétonitrile/H₂O (50/50), acide trifluoroacétique 0,1%. On laisse réagir 5 h sous agitation à température ambiante. Le brut réactionnel est alors purifié par HPLC préparative. Après lyophilisation, on obtient le produit M6G-Glu-SH: [M+H]⁺ = 779 - M_{sel TFA} = 892 - Rdt = 100% - Pureté 95%.

### Exemple 8: synthèse de M6G-(Cys-NBu₂)-S-S-(Cys-NBu₂)-M6G

Couplage de la (Boc-Cys-OH)₂ avec la Di-n-butylamine :

Dans un réacteur (tube falcon) on introduit 1 équivalent molaire de (Boc-Cys-OH)₂ dans du DMF à 105 g/l, 2,2 équivalents molaires de Di-n-butylamine, 2,2 équivalents molaires de 2-(1-H-9-azabenzotriazole-1-yl)-1, 1, 3, 3-tetraméthyluronium hexafluorophosphate (HATU) et 2,2 équivalents molaires de DIEA. On laisse réagir 60 minutes sous agitation à température ambiante. Le groupe protecteur *tert*-butoxycarbonyl est clivé avec un mélange d'acide trifluoroacétique/triisopropylsilane (94/6) à 26 g/l. On laisse sous agitation pendant 3 h à température ambiante. Le milieu est alors dilué dans un mélange acétonitrile/H₂O (50/50) et lyophilisé puis purifié par HPLC préparative.
Après lyophilisation, on obtient le produit (H-Cys-NBu₂)₂: [M+H]⁺ = 463 - M_{sel TFA} = 690 - Rdt = 83% - Pureté 98%.

### Couplage de la M6G :

Dans un réacteur, on introduit 2 équivalents molaires de M6G dihydrate dans du DMF à 99 g/l. On additionne 1 équivalent molaire de produit (H-Cys-NBu₂)₂, 4 équivalents molaires de DIEA et 2 équivalents molaires de PyBOP. On laisse sous agitation à température ambiante pendant 1 h. Le produit brut est alors purifié par HPLC préparative.
Après lyophilisation on obtient le dimère M6G-(Cys-NBu₂)-S-S-(Cys-NBu₂)-M6G: [M+H]⁺ = 1349 - M_{sel TFA} = 1576 - Rdt=36% - Pureté 90%.

### Exemple 9 : synthèse de la M6G-(Cys-NBu₂)-SH

### Réduction du dimère M6G-(Cys-NBu₂)-S-S-(Cys-NBu₂)-M6G:

Dans un réacteur (tube falcon) on introduit 1 équivalent molaire de M6G-(Cys-NBu₂)-S-S-(Cys-NBu₂)-M6G et 3 équivalents molaires de TCEP à 85 g/l d'un mélange acétonitrile/H₂O (50/50), acide trifluoroacétique 0,1%.
Après 4 h de réaction sous agitation à température ambiante, le brut réactionnel est purifié par HPLC préparative.
Après lyophilisation, on obtient le produit M6G-(Cys-NBu₂)-SH: [M+H]⁺ = 676 - M_{sel TFA} = 789 - Rdt=85.8% - Pureté 93%.

### Exemple 10: synthèse de la M6G-Cys-OMe

Dans un réacteur (tube falcon) on introduit 2 équivalents molaires de cystine diméthyl ester sous forme chlorhydrate dans du DMF à 68 g/l et 4 équivalents molaires de DIEA. On laisse sous agitation pendant 2 h à température ambiante. On additionne 1 équivalent molaire de M6G dihydrate en poudre et 1,2 équivalents molaires de PyBOP. On laisse réagir pendant 3 h sous agitation à température ambiante. On réduit alors le pont disulfure par 2 équivalents molaires de TCEP à 11,5 g/l dans un mélange H₂O/acide trifluoroacétique 0,1%. On laisse agiter une nuit à température ambiante. Le produit brut est purifié par HPLC préparative.
On obtient après lyophilisation le produit M6G-Cys-OMe : [M+H]⁺ = 579 - M_{sel TFA} = 692 - Rdt = 83% - Pureté 98%.

### Exemple 11 : synthèse de la M6G-Cys-OH

Dans un réacteur on introduit 1 équivalent molaire de M6G-Cys-OMe dans de l'eau à 69 g/l et 3 équivalents molaires d'hydroxyde de lithium. On laisse réagir sous agitation à température ambiante pendant une nuit.
On réduit le pont disulfure du sous-produit d'oxydation par 3 équivalents molaires de TCEP à 30 g/l dans un mélange H₂O/acide trifluoroacétique 0,1%. On laisse sous agitation pendant 30 min à température ambiante. Le produit brut est alors purifié par HPLC préparative.
Après lyophilisation on obtient le produit M6G-Cys-OH : [M+H]⁺ = 565 - M_{sel TFA} = 678 - Rdt = 42% - Pureté 98%.

### B : Etude de l'effet analgésique

Pour cette étude, on a utilisé le test dit de "tail flick ".

Ce test consiste à placer la queue d'une souris devant une source
d'infrarouge à un temps donné après l'administration du produit testé, pris comme temps 0. La lumière est focalisée sur la surface ventrale de la queue de façon à produire une température de surface de 55°C. On mesure alors le temps de latence (temps de réaction) entre l'administration du produit testé et le moment où la souris bouge la queue.

Les composés étudiés, à savoir la M6G, la morphine et les dérivés selon l'invention ont été administrés par voie intraveineuse à des doses de 0,25 à 5 mg/kg équivalents (5 à 10 souris par groupe). Trois mesures ont été faites avant administration du produit testé pour avoir un temps de base. Le temps de latence pour une même souris a été mesuré à différents temps allant de 15 min à 360 min après l'injection du produit. Un temps maximum de 10 s a été choisi comme temps maximum de réaction.

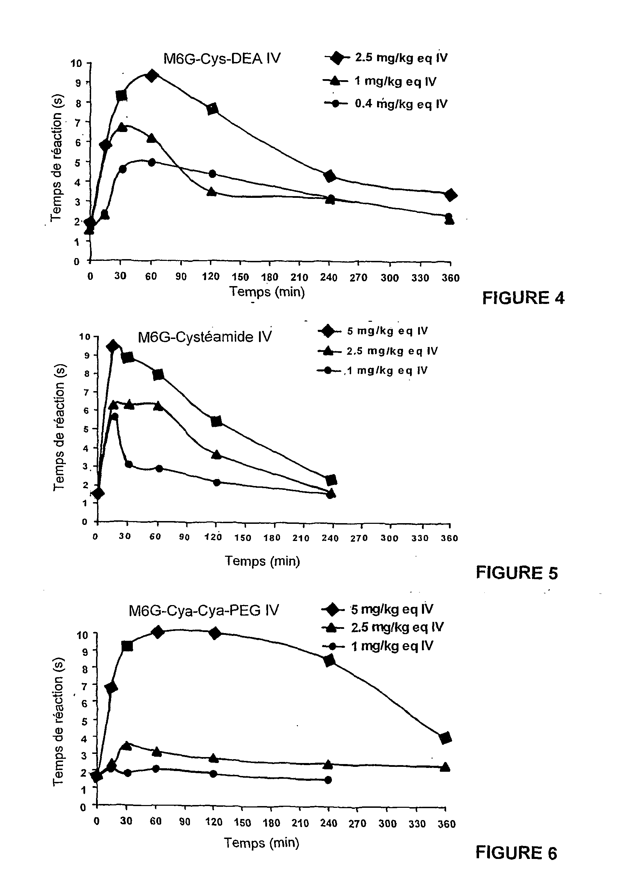

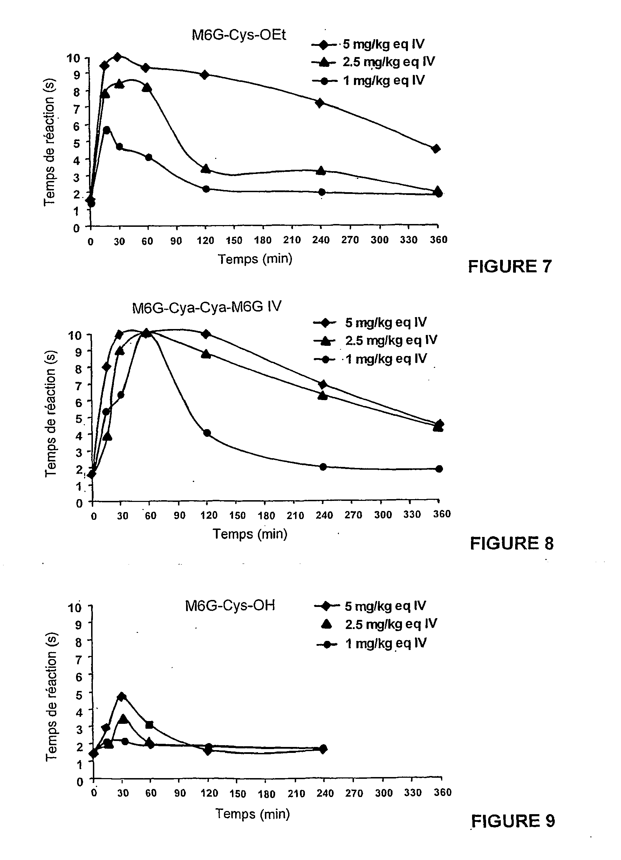

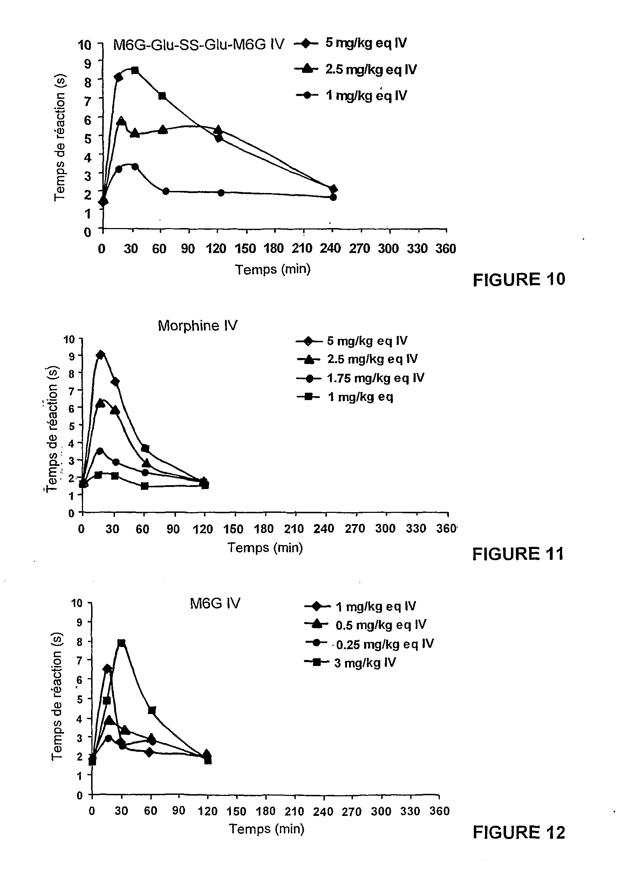

Les résultats obtenus sont représentés par les courbes des figures 4 à 12, sur lesquelles figurent en abscisse, le temps de la mesure (min), et en ordonnée, le temps de réaction (s). Les doses de produits testés sont exprimées en mg équivalents de M6G.

Les symboles suivants sont utilisés dans les figures pour les différents dosages :
- Figure 4 (activité du dérivé M6G-Cys-DEA)
- ◆- 2,5mg /kg eq ; -▲- 1 mg /kg eq ; -●- 0,4 mg /kg eq
- Figure 5 (activité du dérivé M6G-Cystéamide)
- ◆- 5 mg /kg eq ; -▲- 2,5 mg /kg eq ; -●-1 mg /kg eq ;
- Figure 6 (activité du dérivé M6G-Cya-Cya-PEG)
- ◆- 5 mg /kg eq ; -▲- 2,5 mg /kg eq ; -●-1 mg /kg eq ;
- Figure 7 (activité du dérivé M6G-Cys-OEt)
- ◆- 5 mg /kg eq ; -◆- 2,5 mg /kg eq ; -●-1 mg /kg eq
- Figure 8 (activité du dérivé M6G-Cya-Cya-M6G)
- ◆- 5 mg /kg eq ; -▲- 2,5 mg /kg eq ; -●-1 mg /kg eq
- Figure 9 (activité du dérivé M6G-Cys-OH)
- **◆-** 5 mg /kg eq ; -▲- 2,5 mg /kg eq ; -●-1 mg /kg eq
- Figure 10 (activité du dérivé M6G-Glu-SS-Glu-M6G)
- ◆- 5 mg /kg eq ; -▲- 2,5 mg /kg eq ; -●-1 mg /kg eq
- Figure 11 (activité de la morphine)
- ◆- 5 mg /kg eq ; -▲- 2,5 mg /kg eq ; -●-1,75 mg /kg eq
- ■- 1mg/kg
- Figure 12 (activité de la M6G)
- ◆- 1 mg /kg eq ; -▲- 0,5 mg /kg eq ; -●- 0,25 mg /kg eq
- ■- 3 mg/kg

Les résultats montrent que les dérivés de la M6G selon l'invention ont une activité analgésique au moins similaire à la M6G et à la morphine. En effet, la ED₅₀, dose qui induit 50% d'effet analgésique, est comprise entre 0,3 et 2,5 mg éq./kg pour les dérivés selon l'invention, à comparer respectivement à 0,55 et 2,65 pour la M6G et la morphine.

De plus, on observe que pour la plupart des dérivés selon l'invention, l'activité analgésique dure beaucoup plus longtemps. En effet, pour la M6G et la morphine, la durée d'action est d'environ 100 minutes alors que par exemple pour M6G-Cya-Cya-M6G, la durée d'action est de 360 minutes.

### C : étude de l'affinité aux récepteurs opioïdes

### C.1/ Mode opératoire

On a comparé l'affinité de la M6G et de la morphine à celles des dérivés de M6G selon l'invention pour les récepteurs opioïdes µ (mu) et κ (kappa).

Pour déterminer l'affinité aux récepteurs µ, des homogénats de membrane du cortex cérébral du rat (200µg de protéine) ont été incubés avec soit la M6G, ou la morphine ou le composé selon l'invention et 1 nM de [³H][D-Ala², N-MePhe⁴, Gly(ol)⁵]enképhaline (DAMGO) pendant 60 min à 22°C dans un tampon contenant 50 mM Tris-HCl [pH 7,7].

Pour déterminer l'affinité aux récepteurs κ, des homogénats de membrane de cervelet du cobaye (250 µg de protéine) ont été incubés avec soit la M6G, ou la morphine ou le composé selon l'invention et 0,7 nM [³H]U 69593 (80 min à 22°C) dans un tampon contenant 50 mM Tris-HCl [pH 7,4], 10 mM MgCl₂, 1 mM EDTA. On a utilisé des concentrations en M6G, en morphine et en composé selon l'invention de 10⁻¹⁴ à 10⁻⁶ M.

La liaison non spécifique a été déterminée grâce à l'addition aux ligands marqués de naloxone 10 µM.

Après incubation, les échantillons ont été filtrés sur des fibres de verre (GF/B, Packard) préalablement incubées avec 0,3% de polyéthylèneimine et rincées plusieurs fois avec 50 mM de Tris-HCl froid en utilisant un " 96-sample cell harvester " (Unifilter, Packard). Les filtres ont été ensuite séchés et la radioactivité comptée.

### C.2/ Résultats

Les résultats sont rapportés dans le tableau 1 ci-dessous .

**Tableau 1 : Affinité aux récepteurs µ et κ**

| (Ki exprimé en nM). | | |
|---|---|---|
| Composé | récepteurs µ | récepteurs κ |
| Morphine | 12,43 | 155,15 |
| M6G | 13,63 | 223,91 |
| M6G-Cystéamide | 2,35 | 4,49 |
| M6G-Cys-OMe | 1,04 | 14,02 |
| M6G-Cys-OEt | 1,17 | 11,24 |
| M6G-Cys-DEA | 0,36 | 1,90 |
| M6G-Cya-Cya-M6G | 0,38 | 6,22 |
| M6G-Cya-Cya-PEG20000 | 0,92 | 0,72 |

Les résultats montrent que :
- la M6G se lie aux récepteurs µ avec un Ki= de 13,63 nM indiquant une affinité pour ces récepteurs. En revanche, la valeur de Ki pour les récepteurs κ, de l'ordre de 224 nM indique une faible affinité pour ces récepteurs.
- l'affinité des composés selon l'invention pour les récepteurs κ (Ki = 0,72 à 14,02) est augmentée de façon spectaculaire par rapport à celle de la M6G (jusqu'à 310 fois), du fait de la modification chimique, sans que l'affinité pour les récepteurs µ ne diminue . On observe même une augmentation de cette affinité pour les récepteurs µ d'un facteur 10 environ.

## Revendications

1. Composé de formule (A) : 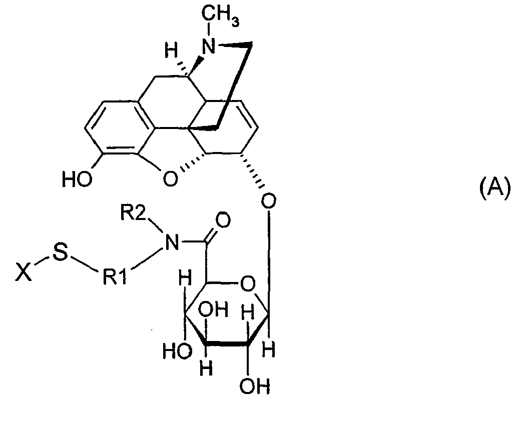 dans laquelle :
- l'ensemble de l'entité ci-dessus, à l'exception du substituant X, est dénommé M6G-N(R₂)R₁-S-
- R₁ représente un groupe alkyle linéaire ou ramifié en C₁-C₁₀, non substitué ou substitué par au moins un substituant, la chaîne alkyle étant éventuellement interrompue par un ou plusieurs hétéroatomes choisis parmi O, SetN;
- R₂ représente l'hydrogène, un groupe alkyle linéaire ou ramifié en C₁-C₅ ou un groupe aryle, hétéroaryle ou (C₁-C₅ )alkylaryle, non substitué ou substitué par un alkyle en C₁-C₄ ;
- X représente l'hydrogène, un résidu M6G-N(R₂)R₁-S- ou un polymère lié au reste de l'entité par un bras espaceur;
- les carbones asymétriques présents dans la formule (A) peuvent être de configuration R ou S,
ainsi que ses sels pharmaceutiquement acceptables.

2. Composé selon la revendication 1, **caractérisé en ce que**
- R₁ et R₂ sont tels que définis dans la revendication 1;
- X représente un résidu M6G-N(R₂)R₁-S-, les deux résidus M6G-N(R₂)R₁-S- constituant les composés de formule (A) sous forme de dimère étant identiques ou différents.

3. Composé selon la revendication 1, **caractérisé en ce que**
- R₁ est tel que défini dans la revendication 1;
- R₂ représente l'hydrogène, et
- X représente l'hydrogène.

4. Composé selon les revendication 1 ou 2, **caractérisé en ce que**
- R₁ est tel que défini dans la revendication 1;
- R₂ représente l'hydrogène, et
- X représente un résidu M6G-N(R₂)R₁-S- dans lequel R₁ et R₂ sont tels que définis ci-dessus.

5. Composé selon l'une quelconque des revendications 1 à 4, **caractérisé en ce que** R₁ représente un groupe alkyle substitué par un ou plusieurs substituants choisi(s) parmi : un groupe alkyle en C₁-C₅; un groupe amino ; un groupe COOR₃ ; un groupe CONR₃R₄ , R₃ et R₄ dans les groupes COOR₃ ou CONR₃R₄ représentant indépendamment l'hydrogène, un groupe alkyle en C₁-C₂₀ éventuellement substitué, aryle, hétéroaryle ou alkylaryle; une cétone en C₁-C₂₀ et un aldéhyde en C₁-C₂₀.

6. Composé selon les revendications 1 ou 3, **caractérisé en ce que** R₁ représente -(CH₂)₂- , R₂ est l'hydrogène et X est l'hydrogène.

7. Composé selon l'une quelconque des revendications 1, 2 ou 4, **caractérisé en ce que** R₁ représente -(CH₂)₂- , R₂ est l'hydrogène et X est un résidu M6G-N(R₂)R₁-S- dans lequel R₁ = -(CH₂)₂- et R₂ est l'hydrogène.

8. Composé selon l'une quelconque des revendications 1, 2 ou 4,
**caractérisé en ce que**
- R₁ représente un groupe -CH(COOR₃)-CH₂- dans lequel R₃ représente l'hydrogène, méthyle, éthyle, propyle ou butyle,
- R₂ représente l'hydrogène,
- X représente l'hydrogène ou un résidu M6G-N(R₂)R₁-S- dans lequel R₁ = -CH(COOR₃)-CH₂- dans lequel R₃ est tel que défini ci-dessus et R₂ est l'hydrogène.

9. Composé selon l'une des revendications 1 ou 5, **caractérisé en ce que**
- R₁ représente un groupe -CH(CONR₃R₄)-CH₂- dans lequel R₃ et R₄ représente l'hydrogène, méthyle, éthyle, propyle ou butyle,
- R₂ représente l'hydrogène,
- X représente l'hydrogène ou un résidu M6G-N(R₂)R₁-S- dans lequel R₁ = -CH(CONR₃R₄)-CH₂- dans lequel R₃ et R₄ sont tels que défini ci-dessus et R₂ est l'hydrogène.

10. Composé selon les revendications 1 ou 5, **caractérisé en ce que**
- R₁ représente un groupe -CH(COOR₃)-C(CH₃)₂- dans lequel R₃ représente l'hydrogène, méthyle, éthyle, propyle ou butyle,
- R₂ représente l'hydrogène
- X représente l'hydrogène ou un résidu M6G-N(R₂)R₁-S- dans lequel R₁ = -CH(COOR₃)-C(CH₃)₂- dans lequel R₃ est tel que défini ci-dessus et R₂ est l'hydrogène.

11. Composé selon les revendications 1 ou 5, **caractérisé en ce que**
- R₁ représente un groupe -CH(COOR₃)-(CH₂)₂-C(O)NHCH(R₅)-CH₂-, dans lequel R₃ représente hydrogène, méthyle, éthyle, propyle ou butyle et R₅ représente -C(O)-NH-CH₂-COOR₃,
- R₂ représente l'hydrogène
- X représente l'hydrogène ou un résidu M6G-N(R₂)R₁-S- dans lequel R₁= -CH(COOR₃)-(CH₂)₂-C(O)NHCH(R₅)-CH₂- dans lequel R₃ et R₅ sont tels que définis ci-dessus et R₂ représente l'hydrogène.

12. Composé selon la revendication 1, **caractérisé en ce que**
- R₁ représente un groupe -(CH₂)₂-,
- R₂ représente l'hydrogène
- X représente un polymère lié au reste de l'entité par un bras espaceur de formule -S-(CH₂)ₙ-NH-C(O)- dans lequel n=0 à 4 et ledit polymère est un polyéthylène glycol de poids moléculaire (Mw) supérieur ou égal à 10000.

13. Procédé de préparation d'un composé de formule (A) selon l'une quelconque des revendications 1 à 12, **caractérisé en ce qu'**il comprend les étapes consistant à faire réagir la morphine-6-glucuronide avec un composé de formule (III) NHR₂-R₁-S-S-R₁-NHR₂, dans laquelle R₁ et R₂ sont tels que définis dans l'une quelconque des revendications 1 à 11, en présence d'un agent de couplage, et à réduire le pont disulfure à l'aide d'un agent réducteur si nécessaire.

14. Procédé de préparation d'un composé de formule (A) selon l'une quelconque des revendications 1 à 11, dans laquelle X = H, **caractérisé en ce qu'**il comprend les étapes consistant à faire réagir la morphine-6-glucuronide avec un composé de formule (IV) NHR₂-R₁-SH, dans laquelle R₁ et R₂ sont tels que définis dans l'une quelconque des revendications 1 à 12, en présence d'un agent de couplage et à réduire *in situ* les sous-produits d'oxydation à l'aide d'un agent réducteur.

15. Procédé selon l'une des revendications 13 ou 14, **caractérisé en ce que** l'agent de couplage est choisi parmi que le benzotriazol-1-yl-oxy-tris-pyrrolidino-phosphonium hexafluorophosphate (PyBOP), la dicyclohexylcarbodiimide (DCC), la DCC associée à l'hydroxybenzotriazole (DCC/HOBT) et la diisopropylcarbodiimide associée à l'HOBT (DIPCDI/ HOBT).

16. Procédé selon l'une des revendications 13 ou 14, **caractérisé en ce que** l'agent réducteur est choisi parmi la tris(2-carboxyéthyl)phosphine, la triphénylphosphine, la tris(hydroxyméthyl)-phosphine et le dithiothréitol.

17. Composition pharmaceutique, **caractérisée en ce qu'**elle contient un composé de formule (A) selon l'une quelconque des revendications 1 à 12 et un véhicule pharmaceutiquement acceptable.

18. Composition pharmaceutique selon la revendication 17, **caractérisée en ce qu'**elle se présente sous une forme administrable par voie parentérale.

19. Composition pharmaceutique selon la revendication 17, **caractérisée en ce qu'**elle se présente sous forme de préparation injectable par voie sous-cutanée, intraveineuse ou intramusculaire.

20. Composition pharmaceutique selon la revendication 19, **caractérisée en ce qu'**elle se présente sous une forme administrable par voie orale.

21. Composition pharmaceutique selon la revendication 20, **caractérisée en ce qu'**elle présente une activité prolongée ou retardée.

22. Utilisation d'un composé selon l'une quelconque des revendications 1 à 12 ou d'une composition pharmaceutique selon l'une quelconque des revendications 17 à 21, pour la fabrication d'un médicament destiné au traitement de la douleur.

## Claims

1. Compound of formula (A): 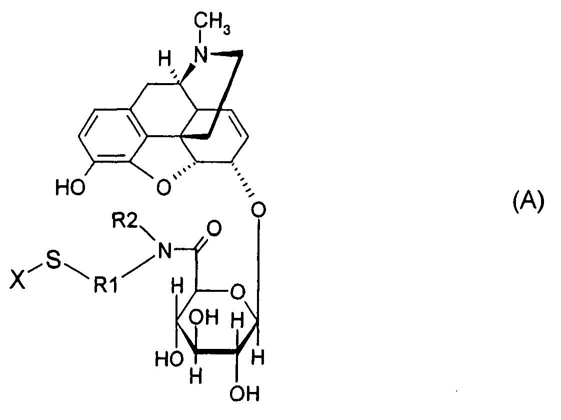 in which:
- all of the above entity, with the exception of the substituent X, is called M6G-N(R₂)R₁-S-
- R₁ represents a linear or branched C₁-C₁₀ alkyl group, unsubstituted or substituted by at least one substituent, the alkyl chain being optionally interrupted by one or more heteroatoms chosen from O, S and N;
- R₂ represents hydrogen, a linear or branched C₁-C₅ alkyl group or an aryl, heteroaryl or (C₁-C₅) alkylaryl group, unsubstituted or substituted by a C₁-C₄ alkyl;
- X represents hydrogen, an M6G-N(R₂)R₁-S- residue or a polymer linked with the rest of the entity by a spacer arm;
- the asymmetric carbons present in the formula (A) can have the R or S configuration,
as well as its pharmaceutically acceptable salts.

2. Compound according to claim 1, **characterized in that**
- R₁ and R₂ are as defined in claim 1;
- X represents an M6G-N(R₂)R₁-S- residue, the two M6G-N(R₂)R₁-S-residues constituting the compounds of formula (A) in dimer form being identical or different.

3. Compound according to claim 1, **characterized in that**
- R₁ is as defined in claim 1;
- R₂ represents hydrogen, and
- X represents hydrogen.

4. Compound according to claim 1 or 2, **characterized in that**
- R₁ is as defined in claim 1;
- R₂ represents hydrogen, and
- X represents an M6G-N(R₂)R₁-S- residue in which R₁ and R₂ are as defined above.

5. Compound according to any one of claims 1 to 4, **characterized in that** R₁ represents an alkyl group substituted by one or more substituents chosen from: a C₁-C₅ alkyl group; an amino group; a COOR₃ group; a CONR₃R₄ group, R₃ and R₄ in the COOR₃ or CONR₃R₄ groups independently representing hydrogen, an optionally substituted C₁-C₂₀ alkyl, an aryl, a heteroaryl or an alkylaryl group; a C₁-C₂₀ ketone and a C₁-C₂₀ aldehyde.

6. Compound according to claims 1 or 3, **characterized in that** R₁ represents -(CH₂)₂-, R₂ is hydrogen and X is hydrogen.

7. Compound according to any one of claims 1, 2 or 4, **characterized in that** R₁ represents -(CH₂)₂-, R₂ is hydrogen and X is an M6G-N(R₂)R₁-S-residue in which R₁= -(CH₂)₂- and R₂ is hydrogen.

8. Compound according to any one of claims 1, 2 or 4, **characterized in that**
- R₁ represents a -CH(COOR₃)-CH₂- group in which R₃ represents hydrogen, methyl, ethyl, propyl or butyl,
- R₂ represents hydrogen,
- X represents hydrogen or an M6G-N(R₂)R₁S- residue in which R₁ = -CH(COOR₃)-CH₂- in which R₃ is as defined above and R₂ is hydrogen.

9. Compound according to one of claims 1 or 5, **characterized in that**
- R₁ represents a -CH(CONR₃R₄)-CH₂- group in which R₃ and R₄ represent hydrogen, methyl, ethyl, propyl or butyl,
- R₂ represents hydrogen,
- X represents hydrogen or an M6G-N(R₂)R₁-S- residue in which R₁ = -CH(CONR₃R₄)-CH₂- in which R₃ and R₄ are as defined above and R₂ is hydrogen.

10. Compound according to claims 1 or 5, **characterized in that**
- R₁ represents a -CH(COOR₃)-C(CH₃)₂- group in which R₃ represents hydrogen, methyl, ethyl, propyl or butyl,
- R₂ represents hydrogen
- X represents hydrogen or an M6G-N(R₂)R₁-S- residue in which R₁ = -CH(COOR₃)-C (CH₃)₂- in which R₃ is as defined above and R₂ is hydrogen.

11. Compound according to claims 1 or 5, **characterized in that**
- R₁ represents a -CH(COOR₃)-(CH₂)₂-C(O)NHCH(R₅)-CH₂- group, in which R₃ represents hydrogen, methyl, ethyl, propyl or butyl and R₅ represents -C(O)-NH-CH₂-COOR₃,
- R₂ represents hydrogen
- X represents hydrogen or an M6G-N(R₂)R₁-S- residue in which R₁= -CH(COOR₃)-(CH₂)₂-C(O)NHCH(R₅)-CH₂- in which R₃ and R₅ are as defined above and R₂ represents hydrogen.

12. Compound according to claim 1, **characterized in that**
- R₁ represents a -(CH₂)₂- group,
- R₂ represents hydrogen
- X represents a polymer linked to the rest of the entity by a spacer arm of formula -S-(CH₂)ₙ-NH-C(O)- in which n = 0 to 4 and said polymer is a polyethylene glycol of molecular weight (Mw) greater than or equal to 10000.

13. Method for the preparation of a compound of formula (A) according to any one of claims 1 to 12, **characterized in that** it comprises the stages consisting of reacting morphine-6-glucuronide with a compound of formula (III) NHR₂-R₁-S-S-R₁-NHR₂, in which R₁ and R₂ are as defined in any one of claims 1 to 11, in the presence of a coupling agent, and reducing the disulphide bridge using a reducing agent if necessary.

14. Method for the preparation of a compound of formula (A) according to any one of claims 1 to 11, in which X = H, **characterized in that** it comprises the stages consisting of reacting morphine-6-glucuronide with a compound of formula (IV) NHR₂-R₁-SH, in which R₁ and R₂ are as defined in any one of claims 1 to 12, in the presence of a coupling agent and reducing *in situ* the oxidation by-products using a reducing agent.

15. Method according to one of claims 13 or 14, **characterized in that** the coupling agent is chosen from benzotriazol-1-yl-oxy-tris-pyrrolidino-phosphonium hexafluorophosphate (PyBOP), dicyclohexylcarbodiimide (DCC), DCC combined with hydroxybenzotriazole (DCC/HOBT) and diisopropylcarbodiimide combined with HOBT (DlPCDl/HOBT).

16. Method according to one of claims 13 or 14, **characterized in that** the reducing agent is chosen from tris(2-carboxyethyl)phosphine, triphenylphosphine, tris(hydroxymethyl)-phosphine and dithiothreitol.

17. Pharmaceutical composition, **characterized in that** it contains a compound of formula (A) according to any one of claims 1 to 12 and a pharmaceutically acceptable vehicle.

18. Pharmaceutical composition according to claim 17, **characterized in that** it is in a form which can be administered by parenteral route.

19. Pharmaceutical composition according to claim 17, **characterized in that** it is in the form of a preparation which can be injected by sub-cutaneous, intravenous or intramuscular route.

20. Pharmaceutical composition according to claim 19, **characterized in that** it is in a form which can be administered by oral route.

21. Pharmaceutical composition according to claim 20, **characterized in that** it has a sustained or controlled activity.

22. Use of a compound according to any one of claims 1 to 12 or a pharmaceutical composition according to any one of claims 17 to 21, for the manufacture of a medicament intended for the treatment of pain.

## Patentansprüche

1. Verbindung der Formel (1): 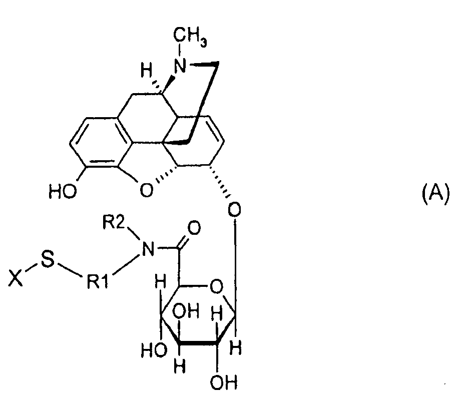 wobei
- die gesamte oben angegebene Einheit mit Ausnahme des Substituenten X als M6G-N(R₂)R₁-S- bezeichnet wird,
- R₁ eine lineare oder verzweigte C₁-C₁₀-Alkylgruppe darstellt, die nicht substituiert oder mit wenigstens einem Substituenten substituiert ist, wobei die Alkylkette gegebenenfalls durch ein oder mehrere Heteroatome, ausgewählt aus O, S und N, unterbrochen ist;
- R₂ Wasserstoff, eine lineare oder verzweigte C₁-C₅-Alkylgruppe oder eine Aryl-, Heteroaryl- oder (C₁-C₅) Alkylaryl-Gruppe, die nicht substituiert oder mit einem C₁-C₄-Alkyl substituiert ist, darstellt;
- X Wasserstoff, einen Rest M6G-N(R₂)R₁-S- oder ein Polymer, über einen Spacearm an den Rest der Einheit gebunden, darstellt;
- die in der Formel (A) vorliegenden asymmetrischen Kohlenstoffatome die R- oder S-Konfiguration haben können,
sowie ihre pharmazeutisch annehmbaren Salze.

2. Verbindung nach Anspruch 1, **dadurch gekennzeichnet, dass**
- R₁ und R₂ wie in Anspruch 1 definiert sind;
- X einen Rest M6G-N(R₂)R₁-S- darstellt, wobei die zwei Reste M6G-N(R₂)R₁-S-, die Verbindungen der Formel (A) in Form eines Dimers bilden, identisch oder unterschiedlich sind.

3. Verbindung nach Anspruch 1, **dadurch gekennzeichnet, dass**
- R₁ wie in Anspruch 1 definiert ist;
- R₂ Wasserstoff darstellt und
- X Wasserstoff darstellt.

4. Verbindung nach Anspruch 1 oder 2, **dadurch gekennzeichnet, dass**
- R₁ wie in Anspruch 1 definiert ist;
- R₂ Wasserstoff darstellt und
- X einen Rest M6G-N(R₂)R₁-S- darstellt, in dem R₁ und R₂ wie oben definiert sind.

5. Verbindung nach einem der Ansprüche 1 bis 4, **dadurch gekennzeichnet, dass** R₁ eine Alkylgruppe darstellt, die mit einem Substituenten oder mehreren Substituenten substituiert ist, der/die ausgewählt ist/sind unter: einer C₁-C₅-Alkylgruppe, einer Aminogruppe, einer COOR₃-Gruppe, einer CONR₃R₄-Gruppe, wobei R₃ und R₄ in den Gruppen COOR₃ oder CONR₃R₄ unabhängig Wasserstoff, eine C₁-C₂₀-Alkylgruppe, die gegebenenfalls substituiert ist, eine Aryl-, Hetaroaryl- oder Alkylarylgruppe darstellen; einem C₁-C₂₀-Keton und einem C₁-C₂₀-Aldehyd.

6. Verbindung nach Anspruch 1 oder 3, **dadurch gekennzeichnet, dass** R₁ -(CH₂)₂- darstellt, R₂ Wasserstoff ist und X Wasserstoff ist.

7. Verbindung nach einem der Ansprüche 1, 2 oder 4, **dadurch gekennzeichnet, dass** R₁
- (CH₂)₂- darstellt, R₂ Wasserstoff ist und X ein Rest M6G-N(R₂)R₁-S- ist, in dem R₁ -(CH₂)₂- ist und R₂ Wasserstoff ist.

8. Verbindung nach einem der Ansprüche 1, 2 oder 4, **dadurch gekennzeichnet, dass**
- R₁ eine Gruppe -CH(COOR₃)-CH₂- darstellt, in der R₃ Wasserstoff, Methyl, Ethyl, Propyl oder Butyl darstellt,
- R₂ Wasserstoff darstellt,
- X Wasserstoff oder einen Rest M6G-N(R₂)R₁-S- darstellt, in dem R₁ -CH(COOR₃)-CH₂- ist, worin R₃ wie oben definiert ist und R₂ Wasserstoff ist.

9. Verbindung nach einem der Ansprüche 1 bis 5, **dadurch gekennzeichnet, dass**
- R₁ eine Gruppe -CH(CONR₃R₄)-CH₂- darstellt, in der R₃ und R₄ Wasserstoff, Methyl, Ethyl, Propyl oder Butyl darstellen,
- R₂ Wasserstoff darstellt,
- X Wasserstoff oder einen Rest M6G-N(R₂)R₁-S- darstellt, in dem R₁ =
- CH(CONR₃R₄)-CH₂-, worin R₃ und R₄ wie oben definiert sind und R₂ Wasserstoff ist.

10. Verbindung nach Anspruch 1 oder 5, **dadurch gekennzeichnet, dass**
- R₁ eine Gruppe -CH(COOR₃)-C(CH₃)₂- darstellt, in der R₃ Wasserstoff, Methyl, Ethyl, Propyl oder Butyl darstellt,
- R₂ Wasserstoff darstellt,
- X Wasserstoff oder einen Rest M6G-N(R₂)R₁-S- darstellt, in dem R₁ =
- CH(COOR₃)-C(CH₃)₂-, worin R₃ wie oben definiert ist und R₂ Wasserstoff ist.

11. Verbindung nach Anspruch 1 oder 5, **dadurch gekennzeichnet, dass**
- R₁ eine Gruppe -CH(COOR₃)-(CH₂)₂-C(O)NHCH(R₅)-CH₂- darstellt, in der R₃ Wasserstoff, Methyl, Ethyl, Propyl oder Butyl darstellt und R₅ -C(O)-NH-CH₂-COOR₃ darstellt,
- R₂ Wasserstoff darstellt,
- X Wasserstoff oder einen Rest M6G-N(R₂)R₁-S- darstellt, in dem R₁ =
- CH(COOR₃)-C(CH₂)₂-C(O)NHCH(R₅)-CH₂-, in dem R₃ und R₅ wie oben definiert sind und R₂ Wasserstoff darstellt.

12. Verbindung nach Anspruch 1, **dadurch gekennzeichnet, dass**
- R₁ eine Gruppe -(CH₂)₂- darstellt,
- R₂ Wasserstoff darstellt,
- X ein Polymer darstellt, das an den Rest der Einheit durch einen Spacerarm der Formel -S-(CH₂)ₙ-NH-C(O)-, worin n=0 bis 4 ist, gebunden ist und das genannte Polymer ein Polyethylenglykol mit einem Molekulargewicht (Mw) über oder gleich 10000 ist.

13. Verfahren zur Herstellung einer Verbindung der Formel (A) nach einem der Ansprüche 1 bis 12, **dadurch gekennzeichnet, dass** es die Stufen umfasst, bestehend aus:
- Umsetzen von Morphin-6-glucuronid mit einer Verbindung der Formel (III) NHR₂-R₁-S-S-R₁-NHR₂, in der R₁ und R₂ wie in einem der Ansprüche 1 bis 11 definiert sind, in Gegenwart eines Kopplungsmittels und
- Reduzieren der Disulfiddrücke mit Hilfe eines Reduktionsmittels, falls erforderlich.

14. Verfahren zur Herstellung einer Verbindung der Formel (A) nach einem der Ansprüche 1 bis 11, in der X = H, **dadurch gekennzeichnet, dass** es die folgenden Stufen umfasst:
- Umsetzen von Morphin-6-glucuronid mit einer Verbindung der Formel (IV) NHR₂-R₁-SH, in der R₁ und R₂, die in einem der Ansprüche 1 bis 11 definiert sind, in Gegenwart eines Kopplungsmittels und
- in situ-Reduzieren der Oxidations-Unterprodukte mit Hilfe eines Reduktionsmittels.

15. Verfahren nach Anspruch 13 oder 14, **dadurch gekennzeichnet, dass** das Kopplungsmittel ausgewählt ist unter Benzotriazo-1-yl-oxy-tris-pyrrolidino-phosphonium-hexafluorphosphat (PyBOP), Dicyclohexylcarbodiimid (DCC), DCC, assoziiert mit Hydroxybenzotriazol (DCC/HOBT) und Diisopropylcarbodiimid, assoziiert mit HOBT (DIPCDI/HOBT).

16. Verfahren nach Anspruch 13 oder 14, **dadurch gekennzeichnet, dass** das Reduktionsmittel ausgewählt ist unter Tris(2-carboxyethyl)phosphin, Triphenylphosphin, Tris(hydroxymethyl)-phosphin und Dithiothreitol.

17. Pharmazeutische Zusammensetzung, **dadurch gekennzeichnet, dass** sie eine Verbindung der Formel (A) nach einem der Ansprüche 1 bis 12 und ein pharmazeutisch annehmbares Vehikel enthält.

18. Pharmazeutische Zusammensetzung nach Anspruch 17, **dadurch gekennzeichnet, dass** sie in einer auf parenteralem Weg verabreichbaren Form vorliegt.

19. Pharmazeutische Zusammensetzung nach Anspruch 17, **dadurch gekennzeichnet, dass** sie in Form einer Präparation vorliegt, die auf subkutanem, intravenösem oder intramuskulärem Weg verabreichbar ist.

20. Pharmazeutische Zusammensetzung nach Anspruch 19, **dadurch gekennzeichnet, dass** sie in einer Form vorliegt, die auf oralem Weg verabreichbar ist.

21. Pharmazeutische Zusammensetzung nach Anspruch 20, **dadurch gekennzeichnet, dass** sie eine verlängerte oder verzögerte Aktivität aufweist.

22. Verwendung einer Zusammensetzung nach einem der Ansprüche 1 bis 12 oder einer pharmazeutischen Zusammensetzung nach einem der Ansprüche 17 bis 21 für die Herstellung eines Medikaments, das zur Behandlung von Schmerzen bestimmt ist.
